# EUROPÄISCHE PATENTSCHRIFT

(11) **EP 0 449 099 B1**
(45) Veröffentlichungstag und Bekanntmachung des Hinweises auf die Patenterteilung: **29.11.1995**
(21) Anmeldenummer: 91104375.0
(22) Anmeldetag: 20.03.1991
(51) Int. Cl.: C07C 403/20, A61K 31/215, A61K 31/075

(54) **Neue Retinoide, ihre Herstellung und Verwendung für Heilmittel**
Retinoide derivatives, their preparation and their use for medicaments
Dérivés rétinoides, leur préparation et leur utilisation dans médicaments

(30) Priorität: 27.03.1990 CH 1008/90
(43) Veröffentlichungstag der Anmeldung: 02.10.1991
(73) Patentinhaber: F. HOFFMANN-LA ROCHE AG, 4002 Basel (CH)
(72) Erfinder: Gross, Günter, Dr., W-7858 Weil a/Rhein (DE)
(74) Vertreter: Lederer, Franz, Dr.

(56) Entgegenhaltungen:
- EP-A- 0 263 492
- US-A- 4 677 120

## Beschreibung

Die vorliegende Erfindung betrifft das neue Retinoid Aethylidenacetat 3,7-dimethyl-9-(2,6,6-trimethyl-1-cyclohexen-1-yl)-2(Z),5,7,8(E)-nonatetraenoat, seine Herstellung und Verwendung zur Herstellung von Heilmitteln, sowie Heilmittel, die diese Verbindung als Wirkstoff enthalten.

Das Aethylidenacetat 3,7-dimethyl-9-(2,6,6-trimethyl-1-cyclohexen-1-yl)-2(Z),5,7,8(E)-nonatetraenoat kann dadurch erhalten werden, dass man 13-cis-Retinsäure oder ein Salz davon mit einer Verbindung der Formel

XCH(CH₃)OC(O)CH₃ II

wobei X Halogen ist umsetzt.

Beispiele für durch X dargestellte Halogene sind insbesondere Chlor oder Jod. Beispiele für Salze der 13-cis-Retinsäure sind Alkalisalze, wie das Natrium- oder das Kaliumsalz; oder Trialkylammoniumsalze, z.B. das Triäthylammoniumsalz; oder, vorzugsweise, Salze mit 1,8-Dicyclo[5.4.0]undec-7-en.

Die Umsetzung wird zweckmässig in einem inerten organischen Lösungsmittel, z.B. in Acetonitril, Dimethylformamid oder Aceton vorgenommen. Die Reaktionstemperatur ist nicht kritisch. Die Reaktion kann bei Raumtemperatur oder unter Erwärmen, z.B. auf Rückflusstemperatur des verwendeten Lösungsmittels vorgenommen werden.

Die Verbindungen der Formeln II sind bekannt oder können in an sich bekannter Weise hergestellt werden. Beispielsweise können die Verbindungen II durch Umsetzung eines Säurechlorids mit einem Aldehyd in Gegenwart von Zinkchlorid (J. Am. Chem. Soc. 43 (1921), 660) hergestellt werden.

Die neue Verbindung ist therapeutisch wirksam. Sie kann beispielsweise zur Behandlung von dermatologischen Erkrankungen verwendet werden, insbesondere bei Akne und solchen, die mit Verhornungsstörungen der Haut einhergehen, wie z.B. Psoriasis, Ichthyosis und Morbus Darier und bei Störungen der Fibroblastenaktivitäten, wie z.B. Keloidose und lokalisierte Sklerodermie; sowie bei Präkanzerosen der Haut.

Strukturell ähnliche Verbindungen sind in US-A-4677120 und EP-A-0263492 als wirksam zur Behandlung von dermatologischen Erkrankungen, z.B. Akne beschrieben.

Die neue Verbindung eignet sich insbesondere zur topischen Anwendung. Im Gegensatz zu den bekannten Verbindungen zeigt sie eine gute Hautverträglichkeit, gute Penetrationsfähigkeit und bewirkt keine oder nur geringe systemische Retinoideffekte.

Die Wirksamkeit der Verbindung kann an Mäusen geprüft, werden, bei denen durch Behandlung mit Dimethylbenzanthracen und Krotonöl Papillome der Haut erzeugt wurden. Durch topische Verabreichung der Verbindung wird eine Rückbildung der Papillome beobachtet, die ein Mass für die therapeutische Wirksamkeit der Verbindungen, z.B. für die Behandlung der Psoriasis, darstellt. Die Versuchsmethodik zur Erzeugung der Papillome ist in Europ. J. Cancer, Vol. 10, 731-737 (1974) beschrieben. Die Papillome wurden während 3 Wochen topisch mit unterschiedlich konzentrierten Lösungen der Versuchsverbindungen behandelt. Dabei wurden von jedem Tier nur 3 Papillome mit der Versuchsverbindung behandelt. Die übrigen Papillome wurden nur mit dem Vehikel behandelt. Eine Beeinflussung dieser, nicht mit den Testverbindungen topisch behandelten Papillome ist sodann auf den systemischen Effekt des topisch verabreichten Versuchspräparats zurückzuführen. Die erhaltenen Resultate sind in Tabelle I angegeben.

Die neue Verbindung kann auch zur Behandlung der alternden Haut verwendet werden.

**Tabelle I**

| **Verbindung** | **Konzentration der applizierten Lösung [%]** | **Veränderung der Papillome [%]** ***** | |
|---|---|---|---|
| | | **behandelt** | **unbehandelt** |
| Vehikel (Aceton) | - | +25 | 0 |
| erfindungsgemässe Verbindung | 0,129 | 0 | 0 |
| | 1,24 | -20 | 0 |
| | 3,2 | -43 | 0 |

| | | | |
|---|---|---|---|
| * Medianwert: mittlerer Wert, in bezug auf den eine jeweils gleiche Anzahl Versuchswerte höher und niedriger ist. | | | |

Ein Modell zur Prüfung der Wirksamkeit bei der Behandlung der Akne ist die Veränderung der Grösse der Talgdrüsen im Ohr des syrischen Goldhamsters nach topischer Applikation der Versuchssubstanz. In diesem Test wird ein Ohr mit Wirkstofflösung, das andere Ohr mit Vehikel behandelt. Die Veränderung der Grösse der Talgdrüsen wird an Gewebeschnitten durch digitale Planimetrie gemessen. Eine Beeinflussung der Talgdrüsen des nur mit Vehikel behandelten Ohrs ist sodann auf einen systemischen Effekt zurückzuführen. Die mit der neuen Verbindung erhaltenen Resultate sind in Tabelle II aufgeführt.

**Tabelle II**

| **Dosis [»g/Tag]** | **Reduktion des Talgdrüsenquerschnitts [%]** | |
|---|---|---|
| | **Wirkstoff** | **Vehikel** |
| 10 | -41 | -9* |
| 100 | -45 | -16* |

| | | |
|---|---|---|
| * statistisch nicht signifikant | | |

Zur topischen Anwendung wird die neue Verbindung zweckmässig in Form von Salben, Tinkturen, Crèmen, Lösungen, Lotionen, Sprays, Suspensionen und dgl. verwendet Bevorzugt sind Salben und Crèmen sowie Lösungen. Diese zur topischen Anwendung bestimmten Präparate können dadurch hergestellt werden, dass man die Verbindung als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,005-2%ige, vorzugsweise 0,01-1%ige Lösungen, Lotionen, Salben oder Crèmes geeignet.

Den Präparaten kann gegebenenfalls ein Antioxydationsmittel, z.B. Tocopherol, N-Methyl-q-tocopheramin sowie butyliertes Hydroxyanisol oder butyliertes Hydroxytoluol beigemischt sein. Weiterhin können die Präparate andere Wirkstoffe, insbesondere Strahlenschutzmittel wie Silikate, Talk, Titandioxid, Zinkoxid oder Zimtsäurederivate wie <Parsol | enthalten.

Die nachstehenden Beispiele erläutern die Erfindung weiter. Die Temperaturen sind in Celsiusgraden angegeben.

### Beispiel 1

Aus 45 g 3,7-Dimethyl-9-(2,6,6-trimetehyl-1-cyclohexen-1-yl)-2-(Z),4,6,8(E)-nonatetraensäure, 22,8 g 1,8-Diazabicyclo(5,4,0)undec-7-en, 20,4 g 1-Chloräthylacetat und 10 g NaJ wird durch Erwärmen in Acetonitril unter Argon und Licht ausschluss das Aethylidenacetat 3,7-dimethyl-9-(2,6,6-trimethyl-1-cyclohexen-1-yl)-2-(Z),4,6,8(E)-nonatetraenoat hergestellt. Die Kristallisation aus Aethanol ergibt eine erste Fraktion des gewünschten Produktes. Nach Eindampfen der Mutterlauge wird über 350 g Kieselgel mit Methylenchlorid-Petroläther-tert.Butylmethyläther 25:71:4 als Elutionsmittel chromatographiert, wodurch eine weitere Fraktion des Produktes erhalten wird. Die Kristallisation der beiden Fraktionen führt zu 23,5 g gelber Kristalle mit einem Schmelzpunkt von 84-86°. Rf (Kieselgel/Methylenchlorid-Petroläther-tert.Butylmethyläther 62:130:8) 0,42.

### Beispiel 2

Ein Hydrogel kann folgende Zusammensetzung aufweisen:

| | |
|---|---|
| Wirkstoff | 0,2 g |
| Hydroxypropylcellulose | 2,0 g |
| Aethanol | 50,0 g |
| Propylenglykol | 20,0 g |
| butyliertes Hydroxytoluol | 0,05 g |
| D,L-α-Tocopherol | 0,15 g |
| Wasser | ad 100,0 g |

### Herstellung:

Der Wirkstoff, das Antioxydans und das Konservierungsmittel werden in Aethanol gelöst. Nach Beimischen der Propylenglykol-Wasser-Lösung lässt man den Gelbildner Klar ausquellen.

### Beispiel 3

Ein Lipogel kann folgende Zusammensetzung aufweisen:

| | |
|---|---|
| Wirkstoff | 0,1 g |
| D,L-α-Tocopherol | 0,15 g |
| Aerosil 200 | 8,0 g |
| Triglycerid, mittelkettig | ad 100,0 g |

### Herstellung:

Der Wirkstoff und das Antioxydans werden im Triglycerid gelöst. Dann wird der Gelbildner unter Rühren eingearbeitet.

### Beispiel 4

Eine Lösung kann folgende Zusammensetzung aufweisen:

| | |
|---|---|
| Wirkstoff | 0,05 g |
| butyliertes Hydroxytoluol | 0,05 g |
| Aethanol | 50,0 g |
| Polyäthylenglykol 400 | ad 100,0 g |

### Herstellung:

Der Wirkstoff und das Konservierungsmittel werden in Aethanol gelöst. Dieser Lösung wird das Polyäthylenglykol zugesetzt.

### Beispiel 5

Eine Oel-in-Wasser Crème kann folgende Zusammensetzung aufweisen:

| | |
|---|---|
| Wirkstoff | 0,2 g |
| butyliertes Hydroxytoluol | 0,05 g |
| Polyoxyäthylen-sorbitanmonostearat | 6,5 g |
| Cetylalkohol | 10,0 g |
| Vaseline, weiss | 25,0 g |
| Glycerin | 10,0 g |
| Benzoesäure | 0,2 g |
| Wasser | ad 100,0 g |

### Herstellung:

Bei 70-75° arbeitet man den Wirkstoff in die geschmolzene Fettphase ein. Glycerin, der Emulgator und Benzoesäure werden dem Wasser zugegeben. Die beiden Phasen werden bei 70° homogenisiert und unter Homogenisieren auf Raumtemperatur erkalten lassen.

### Beispiele 6

Eine Salbe kann folgende Zusammensetzung aufweisen:

| | |
|---|---|
| Wirkstoff | 0,2 g |
| hydroxyliertes Butyltoluol | 0,05 g |
| D,L-α-Tocopherol | 0,15 g |
| Paraffin, dickflüssig | 40,0 g |
| Vaseline, weiss | 45,0 g |
| Ricinusöl, gehärtet | ad 100,0 g |

### Herstellung:

In die 80° heisse Fettphase arbeitet man den Wirkstoff ein und lässt das Gemisch unter Rühren auf Raumtemperatur abkühlen.

## Patentansprüche (Patentansprüche für folgende(n) Vertragsstaat(en): AT, BE, CH, DK, FR, GB, IT, LI, LU, NL, SE)

1. Aethylidenacetat 3,7-dimethyl-9-(2,6,6-trimethyl-1-cyclohexen-1-yl)-2(Z),5,7,8(E)-nonatetraenoat.

2. Die Verbindung des Anspruchs 1 zur Verwendung als Heilmittel.

3. Verfahren zur Herstellung der Verbindung von Anspruch 1, dadurch gekennzeichnet, dass man 13-cis-Retinsäure oder ein Salz davon mit einer Verbindung der Formel
XCH(CH₃)OC(O)CH₃
wobei X Halogen ist, umsetzt.

4. Pharmazeutische und kosmetische Präparate, enthaltend die Verbindung des Anspruchs 1 und einen pharmazeutischen oder kosmetischen Träger.

5. Verwendung der Verbindung des Anspruchs 1 als Wirkstoff bei der Herstellung von Heilmitteln zur topischen Behandlung von dermatologischen Erkrankungen und von Mitteln zur Behandlung der alternden Haut.

## Patentansprüche (Patentansprüche für folgende(n) Vertragsstaat(en): ES, GR)

1. Verfahren zur Herstellung von Aethylidenacetat 3,7-dimethyl-9-(2,6,6-trimethyl-1-cyclohexen-1-yl)-2(Z),5,7,8(E)-nonatetraenoat.
dadurch gekennzeichnet, dass man 13-cis-Retinsäure oder ein Salz davon mit einer Verbindung der Formel
XCH(CH₃)OC(O)CH₃
wobei X Halogen ist umsetzt.

2. Verfahren zur Herstellung pharmazeutischer Präparate, enthaltend Aethylidenacetat 3,7-dimethyl-9-(2,6,6-trimethyl-1-cyclohexen-1-yl)-2(Z),5,7,8(E)-nonatetraenoat. dadurch gekennzeichnet, dass man die Verbindung mit üblichen Hilfs- und Trägerstoffen in eine pharmazeutische Anwendungsform bringt.

3. Verwendung von Aethylidenacetat 3,7-dimethyl-9-(2,6,6-trimethyl-1-cyclohexen-1-yl)-2(Z),5,7,8(E)-nonatetraenoat. als Wirkstoff bei der Herstellung von Heilmitteln zur topischen Behandlung von dermatologischen Erkrankungen und von Mitteln zur Behandlung der alternden Haut.

## Claims (Claims for the following Contracting State(s): AT, BE, CH, DK, FR, GB, IT, LI, LU, NL, SE)

1. Ethylidene acetate 3,7-dimethyl-9-(2,6,6-trimethyl-1-cyclohexen-1-yl)-2(Z),5,7,8(E)-nonatetraenoate.

2. The compound of claim 1 for use as a medicament.

3. A process for the manufacture of the compound of claim 1, characterized by reacting 13-cis-retinoic acid or a salt thereof with a compound of the formula
XCH(CH₃)OC(O)CH₃
wherein X is halogen.

4. Pharmaceutical and cosmetic preparations containing the compound of claim 1 and a pharmaceutical or cosmetic carrier.

5. The use of the compound of claim 1 as the active substance in the manufacture of medicaments for the topical treatment of dermatological disorders and of agents for the treatment of aged skin.

## Claims (Claims for the following Contracting State(s): ES, GR)

1. A process for the manufacture of ethylidene acetate 3,7-dimethyl-9-(2,6,6-trimethyl-1-cyclohexen-1-yl)-2(Z),5,7,8(E)-nonatetraenoate, characterized by reacting 13-cis-retinoic acid or a salt thereof with a compound of the formula
XCH(CH₃)OC(O)CH₃
wherein X is halogen.

2. A process for the production of pharmaceutical preparations containing ethylidene acetate 3,7-dimethyl-9-(2,6,6-trimethyl-1-cyclohexen-1-yl)-2(Z),5,7,8(E)-nonatetraenoate, characterized by bringing the compound into a pharmaceutically administerable form with usual adjuvants and carriers.

3. The use of ethylidene acetate 3,7-dimethyl-9-(2,6,6-trimethyl-1-cyclohexen-1-yl)-2(Z),5,7,8(E)-nonatetraenoate as the active substance in the manufacture of medicaments for the topical treatment of dermatological disorders and of agents for the treatment of aged skin.

## Revendications (Revendications pour l'(les) Etat(s) contractant(s) suivant(s): AT, BE, CH, DK, FR, GB, IT, LI, LU, NL, SE)

1. L'éthylidène acétate du 2(Z)5,7,8(E)-nonatétraénoate de 3,7-diméthyl-9(2,6,6-triméthyl-1-cyclohexène-1-yle).

2. Le composé de la revendication 1 pour l'utilisation en tant que médicament.

3. Procédé de préparation du composé de la revendication 1, caractérisé en ce que l'on fait réagir l'acide 13-cis-rétinoïque ou l'un de ses sels avec un composé de formule
X CH(CH₃)OC(O)CH₃,
dans laquelle X représente un halogène.

4. Compositions pharmaceutiques et cosmétiques contenant le composé de la revendication 1 et un véhicule pharmaceutique ou cosmétique.

5. Utilisation du composé de la revendication 1 en tant que substance active à la préparation de médicaments pour le traitement topique de maladies dermatologiques et de produits pour le traitement contre le vieillissement de la peau.

## Revendications (Revendications pour l'(les) Etat(s) contractant(s) suivant(s): ES, GR)

1. Procédé de préparation de l'éthylidène acétate du 2(Z)5,7,8(E)-nonatétraénoate de 3,7-diméthyl-9-(2,6,6-triméthyl-1-cyclohexène-1-yle), caractérisé en ce que l'on fait réagir l'acide 13-cis-rétinoïque ou l'un de ses sels avec un composé de formule
XCH(CH₃)OC(O)CH₃
dans laquelle X représente un halogène.

2. Procédé de préparation de compositions pharmaceutiques contenant l'éthylidène acétate du 2(Z)5,7,8(E)-nonatétraénoate de 3,7-diméthyl-9-(2,6,6-triméthyl-1-cyclohexène-1-yle), caractérisé en ce que l'on met ce composé sous une forme d'administration pharmaceutique avec des produits auxiliaires et véhicules usuels.

3. Utilisation de l'éthylidène acétate du 2(Z)5,7,8(E)-nonatétraénoate de 3,7-diméthyl-9-(2,6,6-triméthyl-1-cyclohexène-1-yle) en tant que substance active à la préparation de médicaments pour le traitement topique des maladies dermatologiques et de produits pour les traitements contre le vieillissement de la peau.
